# EUROPEAN PATENT APPLICATION

(11) **EP 2 856 963 A1**
(43) Date of publication of application: **08.04.2015**
(21) Application number: 13797743.5
(22) Date of filing: 31.05.2013
(51) Int. Cl.: A61B 18/12, A61B 18/04

(54) **ENERGY-USING TREATMENT TOOL**

(30) Priority: 01.06.2012 US 201261654346 P
(71) Applicant: Olympus Medical Systems Corp., Tokyo 151-0072 (JP)
(72) Inventor: SOBAJIMA, Hideo, Hachioji-shi Tokyo 192-8512 (JP); TAKASHINO, Tomoyuki, Hachioji-shi Tokyo 192-8512 (JP)
(74) Representative: Schicker, Silvia
(86) International application number: PCT/JP2013/065260
(87) International publication number: WO 2013/180293

(57) **Abstract**

A treatment portion of a treatment device includes a pair of jaws, an energy emitter configured to emit energy to the living tissue of the treatment target, an insulating portion which is provided between the jaws and the energy emitter and which has electric insulation to cut off the flow of electricity from the energy emitter to the jaws, an outer edge which is provided in the jaw; a flow path formed by the cooperation of the side of one of the jaws that is provided with the energy emitter facing the other jaw, the energy emitter, the insulating portion, and the outer edges, fluid emanating from the living tissue of the treatment target by the energy emitted from the energy emitter flowing into the flow path, and a support portion which is disposed in the flow path to cover at least a part of the flow path while maintaining an entrance to allow the fluid to flow into the flow path and which supports the living tissue to prevent the peripheral tissues of the living tissue of the treatment target from coming into the flow path.

## Description

### Technical Field

This invention relates to a treatment device to apply energy to a living tissue of a treatment target to treat the living tissue.

### Background Art

For example, US 2010/0042101 A1, US 2008/0195091 A1, US 2009/0270852 A1, and Jpn. Pat. Appln. KOKAI Publication No. 2003-79633 have each disclosed a treatment device which has an openable and closable treatment portion and which can pinch a living tissue in the treatment portion and treat the living tissue.

In general, when energy is applied to the living tissue held by the treatment device having the openable and closable treatment portion to treat the living tissue, fluid such as vapor and body fluid emanates from the living tissue. The temperature of the fluid is high. Therefore, if the fluid flows out from the treatment portion to outside, the living tissue is thermally damaged. Thus, in the treatment portion of the treatment device disclosed in, for example, US 2010/0042101 A1, US 2008/0195091 A1, and US 2009/0270852 A1, a groove is formed in the treatment portion around the outer edge of an energy emitter such as an electrode so that the fluid flows into a space formed by the groove.

However, if the width of the groove is increased so that more fluid flows in, the living tissue comes into the groove when the treatment portion is closed to hold the treatment portion, and the inflow of the fluid may be easily prevented.

On the other hand, if the groove is eliminated from the treatment portion to prevent the living tissue from coming into the groove, the heated fluid may easily escape to the outside of the treatment portion.

### Summary of Invention

An object of this invention is to provide a treatment device which has a groove that allows sufficient inflow of fluid emanating from a living tissue when the living tissue is held by an openable and closable treatment portion and treated by the application of energy to the living tissue, wherein the treatment device can prevent the living tissue from being disposed in the groove when the treatment portion is opened after the treatment of the living tissue.

According to one embodiment of the present invention, a treatment device which applies energy to a living tissue of a treatment target to treat the living tissue and which includes a treatment portion including: a pair of jaws which are relatively openable and closable relative to each other and configured to hold and release the living tissue of the treatment target including peripheral tissues; an energy emitter which is provided in at least one of the pair of jaws and which is configured to emit energy to the living tissue of the treatment target; an insulating portion which is provided between the jaws and the energy emitter and which has electric insulation to cut off the flow of electricity from the energy emitter to the jaws; an outer edge which is provided in the jaw; a flow path formed by the cooperation of the side of one of the jaws that is provided with the energy emitter facing the other jaw, the energy emitter, the insulating portion, and the outer edges, fluid emanating from the living tissue of the treatment target by the energy emitted from the energy emitter flowing into the flow path; and a support portion which is disposed in the flow path to cover at least a part of the flow path while maintaining an entrance to allow the fluid to flow into the flow path and which supports the living tissue to prevent the peripheral tissues of the living tissue of the treatment target from coming into the flow path.

### Brief Description of Drawings

FIG. 1 is a schematic diagram showing a treatment system using energy according to a first embodiment;
FIG. 2 is a schematic block diagram of the medical treatment system according to the first embodiment;
FIG. 3 is a schematic diagram showing how energy is output from a high-frequency energy output circuit and a heat generating component driving circuit of an energy source of the treatment system according to the first embodiment;
FIG. 4A is a schematic perspective view showing how a treatment portion of a treatment device of the treatment system according to the first embodiment is closed;
FIG. 4B is a schematic perspective view showing how the treatment portion of the treatment device of the treatment system according to the first embodiment is opened;
FIG. 5A is a schematic sectional perspective view of a position cut in a surface 5A in FIG. 4A when the treatment portion of the treatment device of the treatment system according to the first embodiment is closed;
FIG. 5B is a schematic sectional perspective view of a position cut in a surface 5B in FIG. 4A when the treatment portion of the treatment device of the treatment system according to the first embodiment is closed;
FIG. 6A is a schematic plan view showing a first treatment portion of the treatment device of the treatment system according to the first embodiment when seen from the side of a second treatment portion;
FIG. 6B is a schematic cross-sectional view of the first treatment portion of the treatment device of the treatment system according to the first embodiment along the line 6B-6B in FIG. 6A;
FIG. 6C is a schematic cross-sectional view of the first treatment portion of the treatment device of the treatment system according to the first embodiment along the line 6C-6C in FIG. 6A;
FIG. 7 is a schematic graph showing, by the comparison between a case with a heat insulating layer (solid line) and a case without a heat insulating layer (broken line), the change of a temperature T relative to a time t in the width-direction center and axial-direction center of a cover 84 when a predetermined output of energy is output from energy emitters to treat a living tissue held by a treatment portion of the treatment device of the treatment system according to the first embodiment;
FIG. 8A is a schematic diagram showing that the treatment device according to the first embodiment is a bipolar type;
FIG. 8B is a schematic diagram showing that the treatment device according to the first embodiment is a monopolar type;
FIG. 9A is a schematic perspective view showing how the treatment portion of the treatment device of the treatment system according to a modification of the first embodiment is closed;
FIG. 9B is a schematic perspective view showing how the treatment portion of the treatment device of the treatment system according to the modification of the first embodiment is opened;
FIG. 10A is a schematic sectional perspective view of a position cut in a surface 10A in FIG. 9A when the treatment portion of the treatment device of the treatment system according to the modification of the first embodiment is closed;
FIG. 10B is a schematic sectional perspective view of a position cut in a surface 10B in FIG. 9A when the treatment portion of the treatment device of the treatment system according to the modification of the first embodiment is closed;
FIG. 11A is a schematic plan view showing the first treatment portion of the treatment device of the treatment system according to the modification of the first embodiment when seen from the side of the second treatment portion;
FIG. 11B is a schematic cross-sectional view of the first treatment portion of the treatment device of the treatment system according to the modification of the first embodiment along the line 11B-11B in FIG. 11A;
FIG. 11C is a schematic cross-sectional view of the first treatment portion of the treatment device of the treatment system according to the modification of the first embodiment along the line 11C-11C in FIG. 11A;
FIG. 12 is a schematic diagram showing a treatment system using energy according to a second embodiment;
FIG. 13A is a schematic longitudinal sectional view showing how a body side treatment portion and a separation side treatment portion of a treatment device of the treatment system according to the second embodiment engage with each other and the separation side treatment portion is separated from the body side treatment portion to open;
FIG. 13B is a schematic longitudinal sectional view showing how the body side treatment portion and the separation side treatment portion of the treatment device of the treatment system according to the second embodiment engage with each other and the separation side treatment portion has been brought into close contact with the body side treatment portion to close;
FIG. 13C is a schematic diagram showing the surface of the body side treatment portion of the treatment device of the treatment system according to the second embodiment;
FIG. 14A is a schematic longitudinal sectional view showing, in an enlarged state, a position indicated by the sign 14A in FIG. 13A in the body side treatment portion of the treatment device of the treatment system according to the second embodiment;
FIG. 14B is a schematic longitudinal sectional view showing, in an enlarged state, a position indicated by the sign 14B in FIG. 13A in the body side treatment portion of the treatment device of the treatment system according to the second embodiment;
FIG. 15A is a schematic longitudinal sectional view showing, in an enlarged state, a position indicated by the sign 15A in FIG. 13A in the separation side treatment portion of the treatment device of the treatment system according to the second embodiment; and
FIG. 15B is a schematic longitudinal sectional view showing, in an enlarged state, a position indicated by the sign 15B in FIG. 13A in the separation side treatment portion of the treatment device of the treatment system according to the second embodiment.

### Brief Description of Embodiments

Hereinafter, embodiments of this invention will be described with reference to the drawings.

### [First Embodiment]

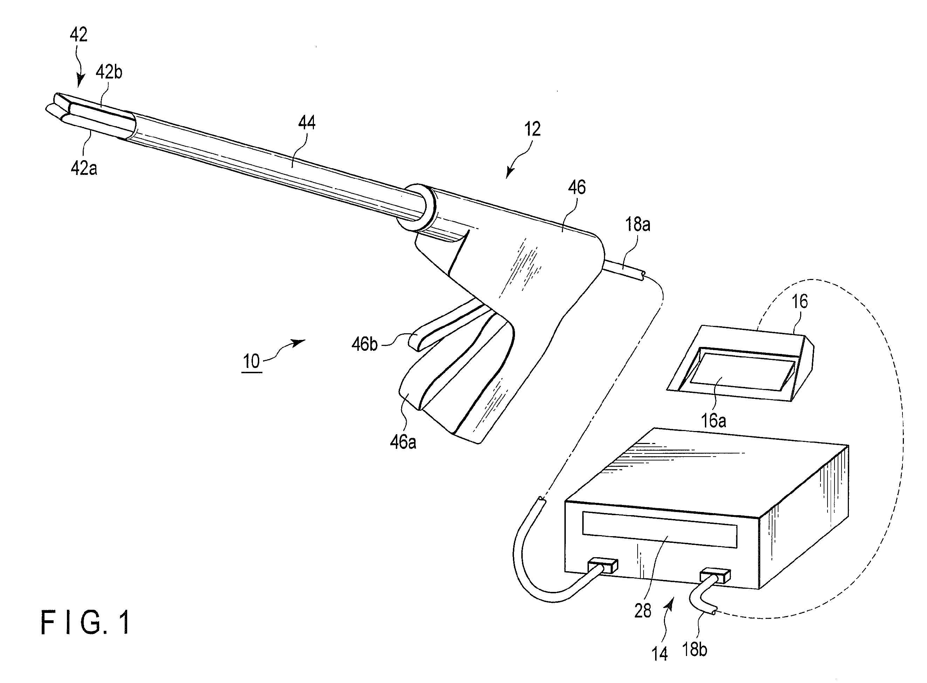

The first embodiment is described with reference to FIG. 1 to FIG. 7.

As shown in FIG. 1, a treatment system 10 using energy according to this embodiment includes a treatment device (energy treatment device) 12, and an energy source 14 which applies energy to the treatment device 12. A foot switch 16 having a pedal 16a to switch on or off energy to be applied to the treatment device 12 is connected to the energy source 14. The treatment device 12 and the energy source 14 are electrically connected to each other by a first cable 18a in which lead wires and signal wires are bundled together. The energy source 14 and the foot switch 16 are electrically connected to each other by a second cable 18b in which lead wires and signal wires are bundled together. The foot switch 16 can input a signal to the energy source 14, for example, by the operation of the pedal 16a. The energy source 14 can control the energy to be applied to the treatment device 12 in accordance with, for example, the operation of the pedal 16a of the foot switch 16.

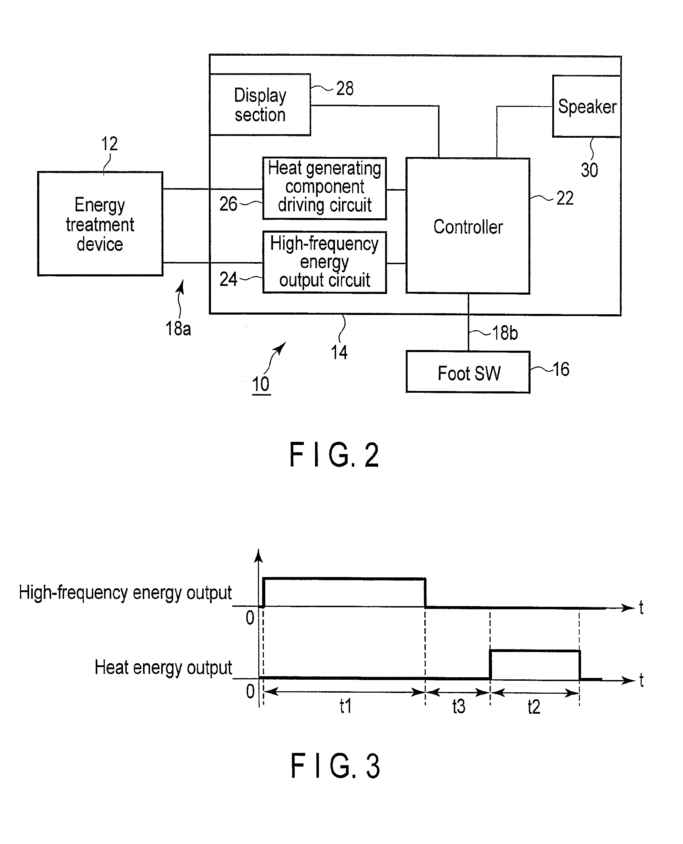

As shown in FIG. 2, the energy source 14 includes a controller 22, a high-frequency energy output circuit 24, a heat generating component driving circuit 26, a display section 28, and a speaker 30.

Here, the high-frequency energy output circuit 24 of the energy source 14 is controlled by the controller 22 to apply high-frequency energy to later-described energy emitters 62 and 64 (electrode 132) of the treatment device 12 so that a living tissue held between the energy emitters 62 and 64 generates heat and is denatured by the heat energy. The heat generating component driving circuit 26 of the energy source 14 is controlled by the controller 22 to supply energy to the energy emitters 62 and 64 (heat generating components (resistance heaters) to generate heat, transmit the heat (heat energy) to the electrode 132, and then transmit the heat (heat energy) to the living tissue to dehydrate the living tissue. That is, the treatment device 12 according to this embodiment applies the heat energy to the living tissue to treat the living tissue.

It is preferable to use, for example, a touch panel as the display section 28 to display the state of the energy source 14 and perform various settings. The speaker 30 is controlled so that the on/off of the output from the high-frequency energy output circuit 24 or the heat generating component driving circuit 26 can be reported by sound.

When applying the high-frequency energy (heat energy) using the later-described energy emitters 62 and 64 (electrode 132) of the treatment device 12 and the heat energy using the later-described energy emitters 62 and 64 (heat generating component 134) to the living tissue, the controller 22 of the energy source 14 can control the supply time of the energy. The controller 22 controls the high-frequency energy output circuit 24 as shown in FIG. 3 in response to the depression of the pedal 16a of the foot switch 16 to output suitable high-frequency energy for a time t1, and then stops the output. The controller 22 then controls the speaker 30 to generate sound so that the end of the treatment using the high-frequency energy is reported to a surgeon. After the treatment using the high-frequency energy, the controller 22 controls the heat generating component driving circuit 26 to output suitable heat energy for a time t2, and then stops the output. The controller 22 then controls the speaker 30 to generate sound so that the end of the treatment using the later-described heat generating component 134 is reported to the surgeon. A time t3 to switch from the treatment using the high-frequency energy to the treatment using the heat energy may be 0, or may be a suitable length of time such as several seconds.

In accordance with the setting in the display section 28, the controller 22 may change from the setting that allows the high-frequency energy output circuit 24 to output the suitable high-frequency energy for the time t1 to the setting such that the high-frequency energy is output by use of the change of bio-information (e.g. impedance and a phase difference) regarding the living tissue which is measurable by the energy emitters 62 and 64. The controller 22 may otherwise stop the output of the high-frequency energy at the time when either one of the two settings (the time and the bio-information) has been reached first.

As shown in FIG. 1, the treatment device 12 includes a treatment portion 42 to treat the living tissue, an insertion portion 44, and an operation portion 46. The treatment portion 42 has a first treatment portion 42a, a second treatment portion 42b, and an open-close operation portion 42c which opens and closes the first and second treatment portions 42a and 42b. The open-close operation portion 42c is a known art and is therefore not described here.

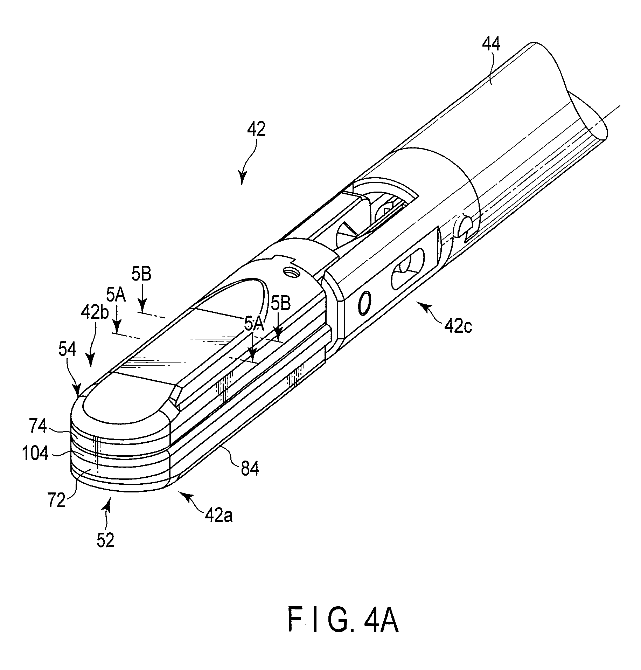

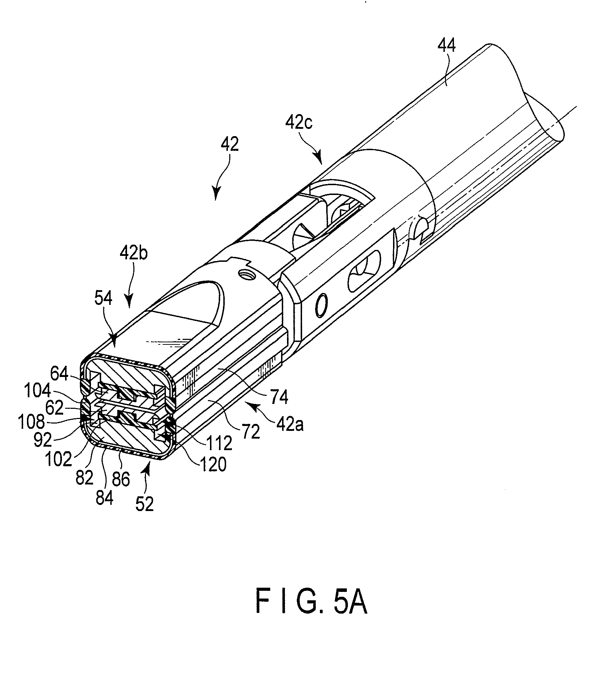

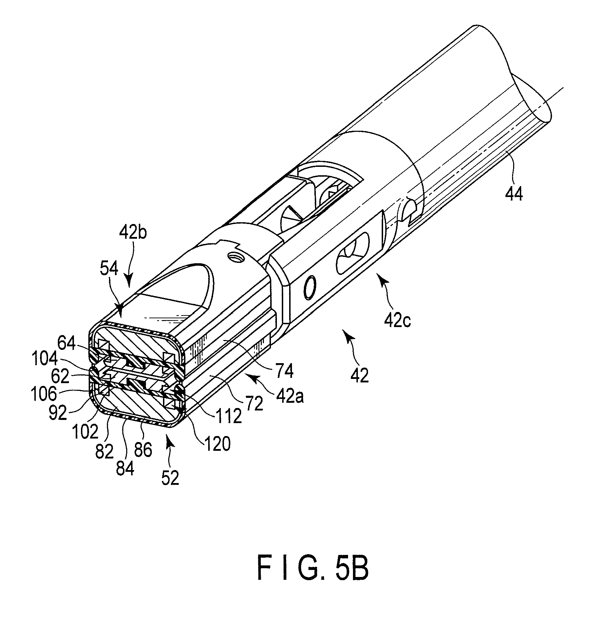

As shown in FIG. 4A to FIG. 5B, the treatment portion 42 includes a pair of openable and closable jaws (first and second jaws) 52 and 54 serving as a holder of the living tissue, the energy emitters (first and second energy emitters) 62 and 64 provided in the jaws 52 and 54, and intermediary portions (first and second intermediary portions) 72 and 74 provided between the jaws 52 and 54 and the energy emitters 62 and 64. Among these portions, the first jaw 52, the first energy emitter 62, and the first intermediary portion 72 form the first treatment portion 42a. Although not described in detail, the second energy emitter 64 and the second intermediary portion 74 provided in the second jaw 54 are preferably formed in the same manner as the first energy emitter 62 and the first intermediary portion 72 provided in the first jaw 52. Thus, the second jaw 54, the second energy emitter 64, and the second intermediary portion 74 form the second treatment portion 42b. That is, although not described in detail, the second treatment portion 42b includes the second jaw 54 formed in the same manner as the first jaw 52, the second energy emitter 64 formed in the same manner as the first energy emitter 62, and the second intermediary portion 74 formed in the same manner as the first intermediary portion 72, as shown in FIG. 5A to FIG. 5B.

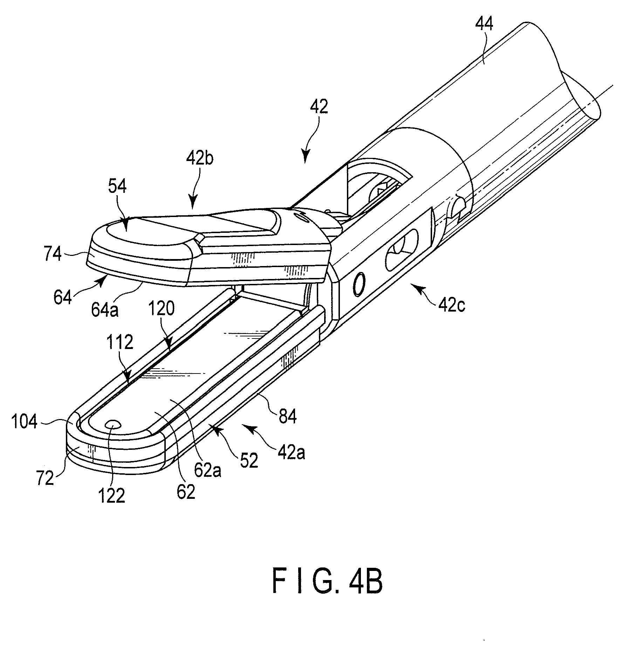

In this embodiment, the energy emitters 62 and 64 are illustrated as being in the form of flat plates in FIG. 4B to FIG. 5B, but are allowed to be in various shapes.

The opening and closing of the first and second jaws 52 and 54 shown in FIG. 4A, that is, the opening and closing of the first and second treatment portions 42a and 42b are operated by an open-close lever 46a of the operation portion 46 shown in FIG. 1. When the open-close lever 46a is operated, the first and second jaws 52 and 54 are opened and closed by known means such as wires and rods disposed in the insertion portion 44. One of the first and second jaws 52 and 54 may be movable, or both of them may be movable. That is, the first and second jaws 52 and 54 can be relatively opened and closed. In the example described according to this embodiment, one (the first jaw 52) of the first and second jaws 52 and 54 is movable relative to the other (the second jaw 54).

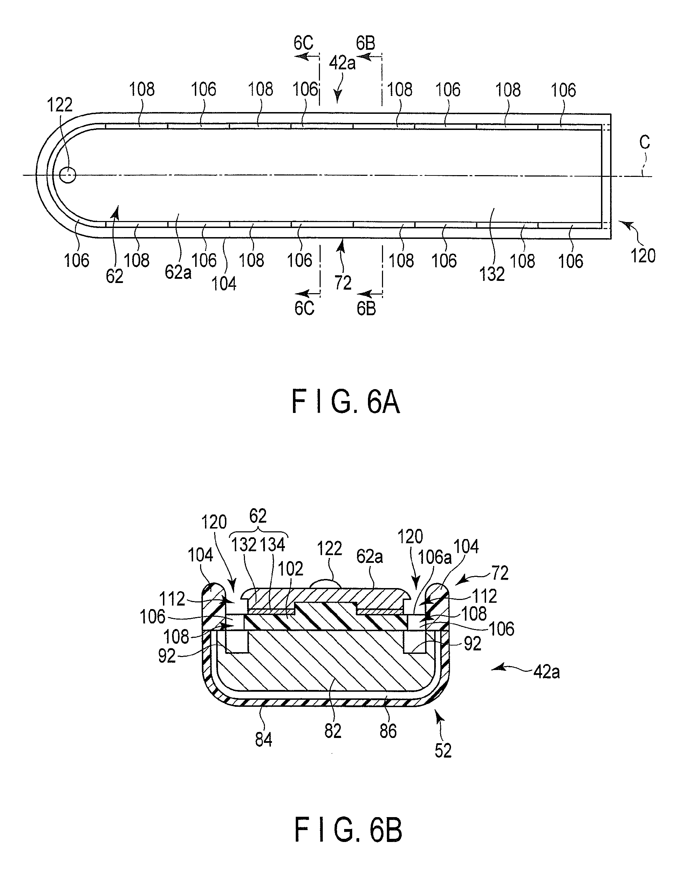

As shown in FIG. 6A to FIG. 6C, the first jaw 52 has a main body 82 and a cover 84. The main body 82 is preferably made of a rigid material such as stainless steel to ensure the force to hold the living tissue. The cover 84 can be made of various materials such as metallic materials, ceramics, and resin materials that have heat resistance and electric insulation.

The cross-section of the main body 82 is substantially rectangular. Depressions (grooves) 92 formed as a part of a later-described flow path 120 are provided in parallel along a longitudinal direction on the side of the main body 82 facing the other jaw 54. The proximal ends of the depressions (first channel) 92 are open, and the depressions 92 are closed at the distal end of the main body 82 or are in communication with each other. The depressions 92 are formed in the vicinity of the outer edge of the main body 82.

The cover 84 is on the side of the main body 82 opposite to the other jaw 54. A heat insulating layer 86 which inhibits the heat conduction from the main body 82 to the cover 84 is formed between the main body 82 and the cover 84. The heat insulating layer 86 is preferably a vacuum, but may contain air or may be filled with various heat insulating materials (preferably materials having heat resistance and electric insulation).

The intermediary portion 72 can be made of various materials such as metallic materials, ceramics, and resin materials that have heat resistance and electric insulation. A part of the intermediary portion 72 is provided between the first energy emitter 62 and the main body 82. The intermediary portion 72 includes a substantially rectangular plate-shaped base (insulating portion) 102 provided between the first energy emitter 62 and the main body 82, an outer edge 104 having abutment portions 104a which respectively abut on the edge of the main body 82 and the edge of the cover 84 of the first jaw 52, and coupling portions (living tissue support portions) 106 which discretely couple the base 102 to the outer edge 104 at a suitable distance. The coupling portions 106 are formed to be disposed on the later-described flow path 120. That is, as shown in FIG. 6A, openings (second channel) 108 are formed between the coupling portions 106 provided along the axial direction. In other words, the coupling portions 106 and the openings 108 are alternately provided on the depressions 92 of the main body 82. Since the openings (spaces) 108 are formed between the coupling portions 106, the fluid emanating from the living tissue can flow to the depressions 92 of the main body 82 from the upper part of the first energy emitter 62 through the openings 108.

The coupling portions 106 can support the living tissue to prevent the held living tissue from coming into the depressions 92 when the living tissue is held between the first and second treatment portions 42a and 42b.

The first energy emitter 62 is disposed in a region between the depressions 92 of the main body 82. A space (third channel) 112 is formed between the first energy emitter 62 and the outer edge 104 of the intermediary portion 72. Therefore, the fluid emanating from the living tissue can flow to the space 112 between the first energy emitter 62 and the outer edge 104 of the intermediary portion 72.

As shown in FIG. 6A to FIG. 6C, the space 112 between the first energy emitter 62 and the outer edges 104 of the intermediary portion 72, the openings 108 between the base 102 of the intermediary portion 72 and the outer edges 104, and the depressions 92 of the main body 82 cooperate to form the flow path 120 of the fluid emanating from the living tissue.

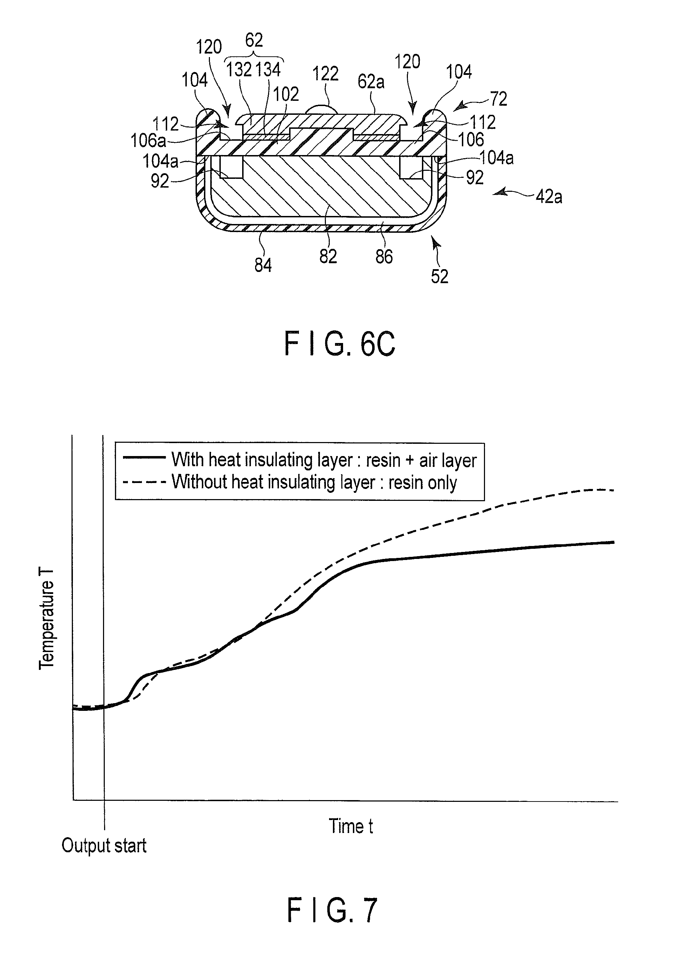

Here, as shown in FIG. 6B and FIG. 6C, the space 112 to pass the fluid emanating from the living tissue is formed between the first energy emitter 62 and the outer edges 104 of the intermediary portion 72. However, since the coupling portions 106 are formed between the base 102 of the intermediary portion 72 and the outer edges 104, the fluid does not flow directly into the depressions 92 of the main body 82 through the coupling portions 106. However, the openings (spaces) 108 are formed between the coupling portions 106, so that the fluid emanating from the living tissue can flow into the depressions 92 of the main body 82 from the coupling portions 106 through the openings (spaces) 108. The sides of the coupling portions 106 located close to the second treatment portion 42b are located far from a surface (living tissue holding surface) 62a of the first energy emitter 62. Therefore, the fluid emanating from the living tissue can flow into the depressions 92 from surfaces 106a of the coupling portions 106 on the side of the space 112 (the other treatment portion 42b) through the openings 108.

The living tissue is disposed and supported on the surfaces 106a of the coupling portions 106 of the intermediary portion 72 when the living tissue is held between the surfaces 62a and 64a of the energy emitters 62 and 64 facing each other and between the outer edges 104 facing each other. This maximally prevents the fluid from coming into the depressions 92 of the main body 82 from the openings 108 of the intermediary portion 72.

As shown in FIG. 6B and FIG. 6C, the energy emitter 62 provided in the first jaw 52 includes the high-frequency electrode 132, and the heat generating component 134 provided in the high-frequency electrode 132. Although not described in detail, the second energy emitter 64 provided in the second jaw 54 is preferably formed in the same manner as the energy emitter 62 provided in the first jaw 52.

For the heat generating component 134, a plurality of heat generating elements may be used, or a plate-shaped heater may be used. If the heat generating component 134 includes a plurality of the heat generating elements, the heat generating elements are preferably disposed on or embedded in the rear surface of the electrode 132. If the heat generating component 134 includes the plate-shaped heater, the plate-shaped heater is preferably disposed on the rear surface of the electrode 132. It is also preferable that the heat generating component 134 has a bar shape which is longer in the longitudinal direction of the electrode 132 or in a direction that intersects at right angles with the longitudinal direction.

A solid line in FIG. 7 represents the change of a temperature T relative to a time t in the surface of the cover 84 (e.g. the width-direction center of the surface of the cover 84, and the axial-direction center) when a predetermined output of energy is output from the energy emitters 62 and 64 to treat the living tissue held by the treatment portion 42 according to the present embodiment. Here, for comparison, a broken line in FIG. 7 represents the temperature change in the surface of the cover 84 (e.g. the width-direction center of the surface of the cover 84, and the axial-direction center) when the heat insulating layer 86 is not provided and the cover 84 made of a resin material having heat resistance and electric insulation is put in close contact with the side of the main body 82 of the first jaw 52 opposite to the second jaw 54.

As seen from FIG. 7, it is more difficult for the temperature to rise when an air layer is formed as the heat insulating layer 86 between the cover 84 and the main body 82 than when the heat insulating layer 86 is not formed. Therefore, it is possible to prevent the treatment portion 42 from thermally affecting the peripheral tissues outside the living tissue of a treatment target.

In the surface 62a of the first energy emitter 62, a protrusion 122 having electric insulation and heat resistance is formed to keep a given distance between the surfaces 62a and 64a of the energy emitters 62 and 64 facing each other when the treatment portion 42 is closed. This prevents the surfaces 62a and 64a of the energy emitters 62 and 64 from contacting each other when the treatment portion 42 is closed. The protrusion 122 may be formed in one of the energy emitters 62 and 64 or may be formed in both of them. The protrusion 122 may be formed in the distal portion of the energy emitter 62 on the distal side relative to the operation portion 46 as shown in FIG. 6A. Although not shown, the protrusion 122 may be formed in the proximal portion of the energy emitter 62 on the proximal side relative to the operation portion 46. The protrusions 122 may be formed in both the distal and proximal portions.

Next, the function of the treatment system 10 which applies energy to a living tissue of a joining target to treat the living tissue according to this embodiment is described.

For example, the treatment portion 42 is put to face a living tissue to be joined. In this condition, the open-close lever 46a of the operation portion 46 is operated to hold the living tissue of the treatment target between the surfaces 62a and 64a of the energy emitters 62 and 64. Thus, when the living tissue of the treatment target and its peripheral tissues are held by the first and second treatment portions 42a and 42b, the living tissue of the treatment target is disposed in close contact with the surfaces 62a and 64a of the energy emitters 62 and 64. The peripheral tissues of the living tissue of the treatment target are held in close contact between the outer edges 104.

When the living tissue is held by the first and second treatment portions 42a and 42b, some of the peripheral tissues of the living tissue of the treatment target come into the space 112 of the first treatment portion 42a. The surfaces 106a of the coupling portions 106 support the provided peripheral tissues so that the peripheral tissues do not come into the depressions 92 from the adjacent openings 108 through the space 112. Thus, when the living tissue is held by the first and second treatment portions 42a and 42b, the peripheral tissues of the living tissue of the treatment target are held in a pressed state on the outer edge 104 of the first treatment portion 42a. At the same time, the part surrounded by the living tissue of the treatment target and its peripheral tissues, the outer edge 104 provided in the first jaw 52, the flow path 120, and the surface 62a of the energy emitter 62 is closed.

When the pedal 16a of the foot switch 16 is kept depressed by a foot in this condition, the controller 22 of the energy source 14 applies energy to the living tissue between the energy emitters 62 and 64 from the high-frequency energy output circuit 24. Thus, heat is generated in the living tissue between the surfaces 62a and 64a of the energy emitters 62 and 64 by heat energy (Joule heat) which has been generated by the high-frequency energy. After the living tissue is denatured by the heat energy, the supply of the energy to the energy emitters 62 and 64 is stopped. The controller 22 of the energy source 14 stops the output of the energy from the high-frequency energy output circuit 24 after energy has been applied to the living tissue between the energy emitters 62 and 64 for the time t1.

Here, when the predetermined time t1 has passed since the start of the output of the energy, the energy source 14 stops the supply of the energy to the energy emitters 62 and 64 even if the pedal 16a of the foot switch 16 is depressed. On the other hand, when the foot is taken off of the pedal 16a before the passage of the predetermined time t1, the energy source 14 stops the supply of the energy to the energy emitters 62 and 64 at the moment when the foot is removed from the pedal 16a.

Thus, when heat is generated in the living tissue of the treatment target between the surfaces 62a and 64a of the energy emitters 62 and 64, fluids such as vapors (gases) and body fluids (liquids) emanate from the living tissue which is in contact or in close contact with the surfaces 62a and 64a. In this case, the part surrounded by the living tissue of the treatment target and its peripheral tissues, the outer edge 104, the flow path 120, and the surface 62a of the first energy emitter 62, is formed as a closed space so that its internal pressure is increased. Thus, the fluid flows toward the space 112 of the flow path 120 along the surface 62a of the first energy emitter 62, that is, the surface of the living tissue of the treatment target, and flows into the space 112.

In this way, the internal pressure of the part surrounded by the living tissue, the outer edge 104, the flow path 120, and the surface 62a of the energy emitter 62 is increased. Thus, part of the fluid will flow out from the inside of the treatment portion 42 along the outer edge 104.

Here, from the surface 62a of the first energy emitter 62, the flow path 120 is formed with a depth which is a combination of the thickness of the first energy emitter 62, the thickness of the base 102 of the intermediary portion 72, and the depth of the depression 92 of the main body 82 of the first jaw 52. That is, the flow path 120 is formed deeper than, for example, when the base 102 is not provided and the thickness of the first energy emitter 62 and the main body 82 only cooperate to form the flow path. Thus, the volume of the flow path 120 can be increased without any change in a direction that intersects at right angles with the axial direction and the width direction of the first treatment portion 42a. This ensures that much of the fluid generated from the living tissue can flow into the farthest depression 92 of the flow path 120 through the space 112 and the openings 108 of the flow path 120.

The flow path is formed deeper in a direction that intersects at right angles with the axial direction and the width direction of the first treatment portion 42a than, for example, when the flow path does not have the base 102 but has the thickness of the first energy emitter 62 and the main body 82. This ensures the fluid which has flowed into the far part (the depression 92) in the flow path 120 does not easily come out of the flow path 120. That is, the flow path 120 is formed with a narrow entrance and with a great depth (depth direction), and this can prevent the fluid from easily coming out of the flow path 120 once the fluid has flowed in the flow path 120. The coupling portions 106 also serve to confine the fluid in the flow path 120. Thus, the coupling portions 106 can further ensure that the fluid is confined in the flow path 120. Therefore, it is possible to maximally prevent the fluid from flowing out of the treatment portion 42.

The peripheral tissues of the living tissue of the treatment target which have come into the space 112 and which are supported by the surface of the coupling portions 106 are more effected by the high-frequency energy particularly in parts located closer to the energy emitter 62 (high-frequency electrode 132). Among the peripheral tissues which have come into the space 112, some of the tissues located close to the energy emitter 62 are treated together with the living tissue of the treatment target. The fluid emanating from the living tissue on the surface 62a of the energy emitter 62 is higher in temperature than the peripheral tissues, and the fluid moves along the surfaces of the living tissue and its peripheral tissues, so that the peripheral tissues which have come into the space 112 and which are supported by the surface of the coupling portions 106 are more easily affected by thermal spread. However, since the peripheral tissues are firmly held by the outer edge 104, the inner edge of the outer edge 104 functions as a barrier to prevent the fluid from moving out. Thus, it is possible to prevent the fluid from flowing into the flow path 120 and flowing out of the treatment portion 42.

If the pedal 16a of the foot switch 16 is kept depressed by a foot, the output of the energy from the high-frequency energy output circuit 24 is stopped, and energy is then output from the heat generating component driving circuit 26 for the time t2 after the passage (which may be zero seconds) of the suitable time (the time t3 in FIG. 3) so that heat is generated in a heat generating component 144. Thus, the heat (heat energy) of the heat generating component 144 is transmitted to the high-frequency electrode 132, and the living tissue can be dehydrated on the surface 62a of the first energy emitter 62. In this case as well, fluid emanates from the living tissue of the treatment target, and the fluid will flow out of the treatment portion 42 via the peripheral tissues. However, the fluid flows into the flow path 120 as described above, and the peripheral tissues are pressed by the outer edges 104 so that the outflow of the fluid is prevented and the thermal spread is inhibited.

In a series of treatments, a similar treatment is performed between the second treatment portion 42b and the living tissue of the treatment target and its peripheral tissues, so that this is not described here.

As described above, the following can be said according to this embodiment.

Since the flow path 120 is formed to be larger in the thickness direction of the first treatment portion 42a, the volume of the flow path 120 can be increased without the increase in the width direction of the first treatment portion 42a. Therefore, more of the fluid emanating from the living tissue by the treatment with energy can flow into the flow path 120, and the emission of the heat to the outside of the treatment portion 42 through the peripheral tissues can be maximally prevented.

When the living tissue of the treatment target and its peripheral tissues are held between the first and second treatment portions 42a and 42b, the peripheral tissues can be held by the coupling portions 106, so that it is possible to prevent the peripheral tissues from coming into the depression 92 through the openings 108.

The heat insulating layer 86 is formed in the jaw 52 according to this embodiment. Therefore, even if the fluid flows into, for example, the farthest depression 92 of the flow path 120 and heat is transmitted to the main body 82 of the first jaw 52, it is possible to maximally prevent the heat coming from the fluid from being released to the outside of the treatment portion 42 in the thickness direction because the heat insulating layer 86 is formed.

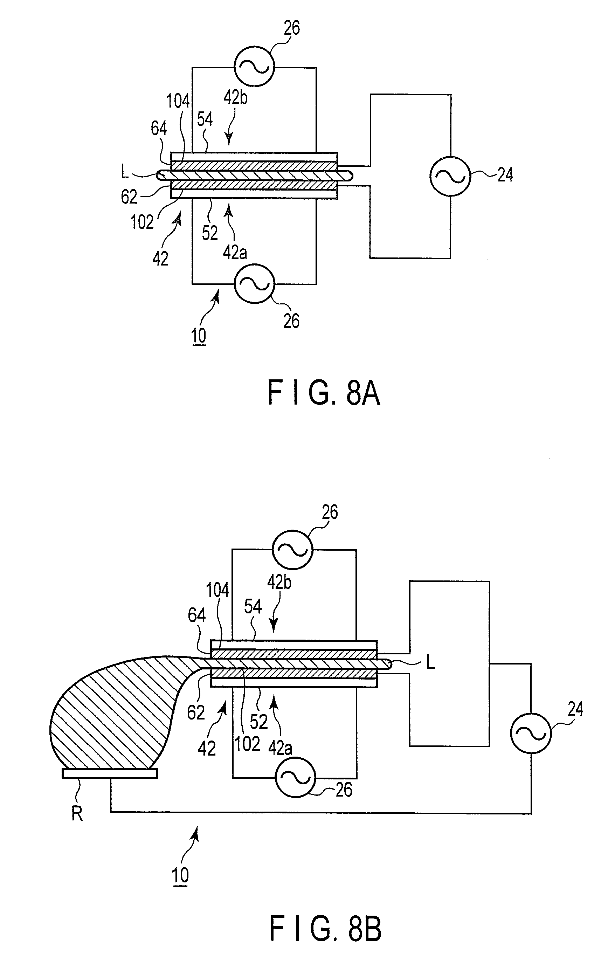

Although the treatment device 12 is a bipolar type treatment device shown in FIG. 8A in the case described according to this embodiment, the treatment device 12 may be used as a monopolar type treatment device shown in FIG. 8B. In the case shown in FIG. 8B, a treatment is conducted with a return electrode R attached to a patient P. That is, the treatment of the living tissue using the energy emitters 62 and 64 may be conducted by either the monopolar type or the bipolar type. When the treatment device 12 according to the present embodiment is used as the monopolar type, high-frequency energy may be applied to only one of the energy emitters 62 and 64 disposed in the pair of jaws 52 and 54. As shown in FIG. 8A and FIG. 8B, it is also preferable to use plate-shaped heaters as the energy emitters 62 and 64.

### [First Modification of First Embodiment]

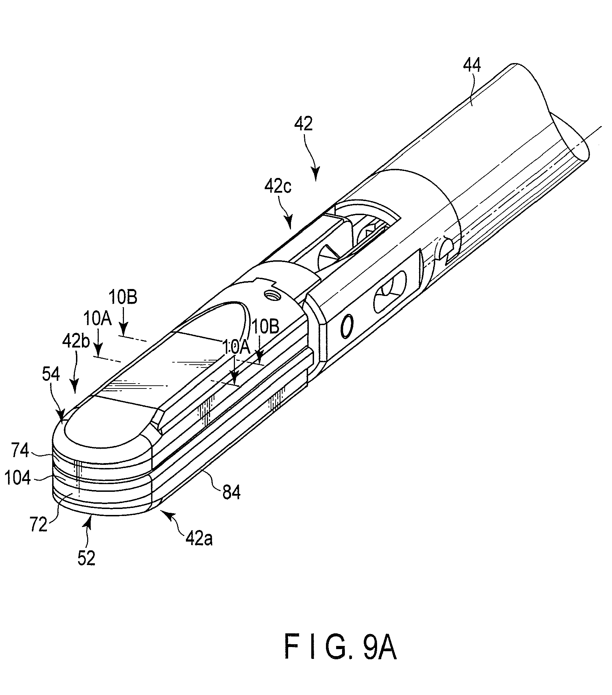

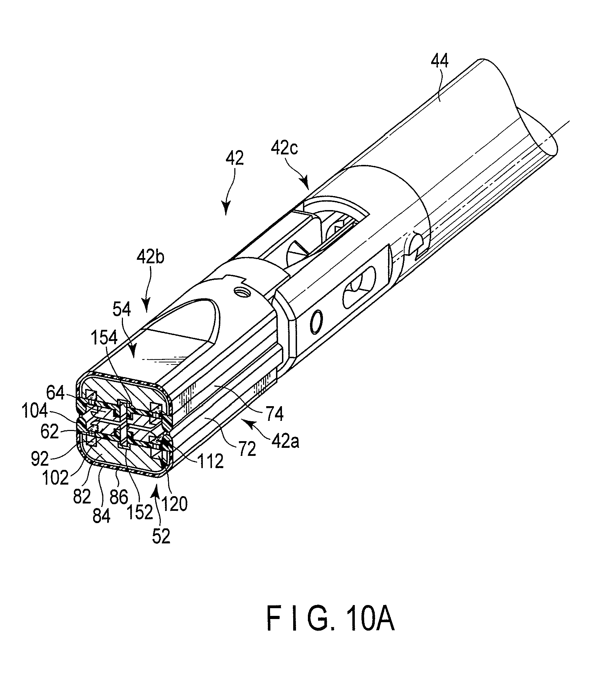

Next, a first modification of the first embodiment is described with reference to FIG. 9A to FIG. 11C. This modification is a modification of the first embodiment.

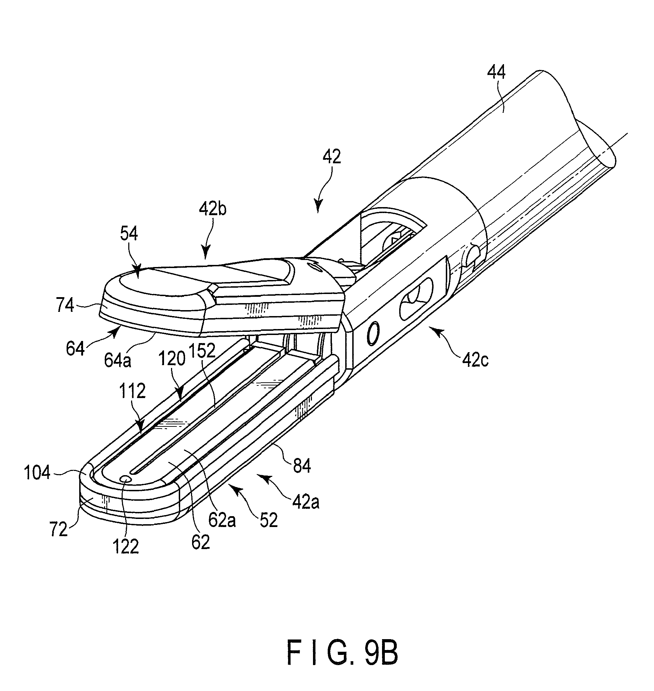

As shown in FIG. 9, the energy emitter 62, the base 102 of the intermediary portion 72, and the main body 82 of the first jaw 52 cooperate to form a cutter guide groove 152 at the position of the first treatment portion 42a facing the second treatment portion 42b. The cutter guide groove 152 has an insulating property. The energy emitter 64, the base 102 of the second intermediary portion 74, and the second jaw 54 cooperate to form a cutter guide groove 154 at the position of the second treatment portion 42b facing the first treatment portion 42a.

An unshown cutter can be inserted into and removed from the cutter guide grooves 152 and 154. The cutter is coupled to a cutter moving lever 46b of the operation portion 46 shown in FIG. 1 via an unshown rod. Thus, the cutter can be guided within a predetermined range along the axial direction of the insertion portion 44 by the operation of the cutter moving lever 46b. That is, the cutter can be moved between a condition in which the edge of the cutter is located at a position between the first and second jaws 52 and 54, and a condition in which the edge of the cutter is retracted in the insertion portion 44 from the space between the first and second jaws 52 and 54. Thus, after the living tissue of the treatment target is treated by the application of energy thereto from the energy emitters 62 and 64, the cutter moving lever 46b is operated to move the edge of the cutter from the proximal side of the first and second jaws 52 and 54 to the distal side of the first and second jaws 52 and 54, so that the living tissue of the treatment target can be cut.

The cutter guide grooves 152 and 154 have the function to let in the fluid emanating from the living tissue in the same manner as the flow path 120.

A cooling pipe (cooling portion) 162 is formed at the position of the outer edge 104 of the first treatment portion 42a which comes in and out of contact with the outer edge 104 of the second intermediary portion 74 of the second treatment portion 42b. A refrigerant can be circulated through the cooling pipe 162. A cooling pipe (cooling portion) 162 through which a refrigerant can be circulated is preferably formed at the position of the outer edge 104 of the second treatment portion 42b which comes in and out of contact with the outer edge 104 of the intermediary portion 72 of the first treatment portion 42a. Thus, for example, the living tissue is held between the holding surfaces 62a and 64a, the peripheral tissues are held between the intermediary portions 72 and 74, and energy is then emitted from the energy emitters 62 and 64. Moreover, the refrigerant is circulated through the cooling pipe 162 to cool the peripheral tissues. As a result, the thermal spread can be more effectively inhibited.

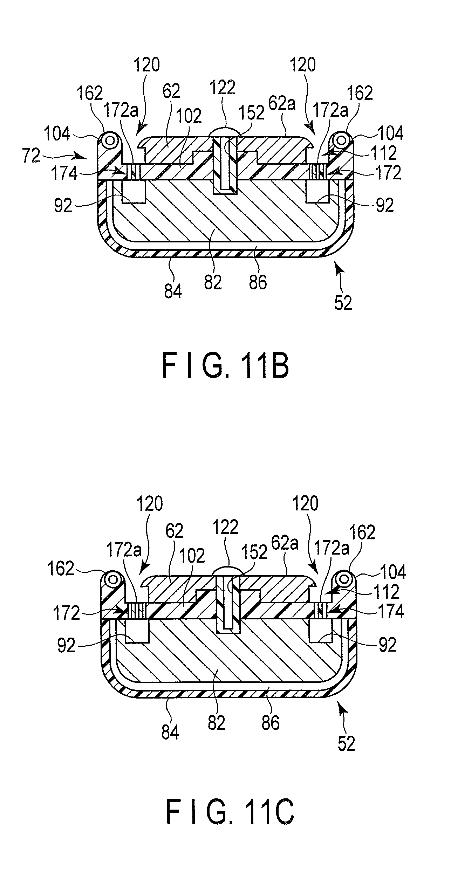

The cooling pipe 162 shown in FIG. 11B and FIG. 11C may not be formed when no energy emitter is provided in the surface 62a.

A plate having satisfactory thermal conduction may be disposed on the outer circumferential surface of the outer edge 104, and the heat of the cooling pipe 162 may be transmitted to the plate to cool the whole peripheral tissues pressed by the outer edge 104.

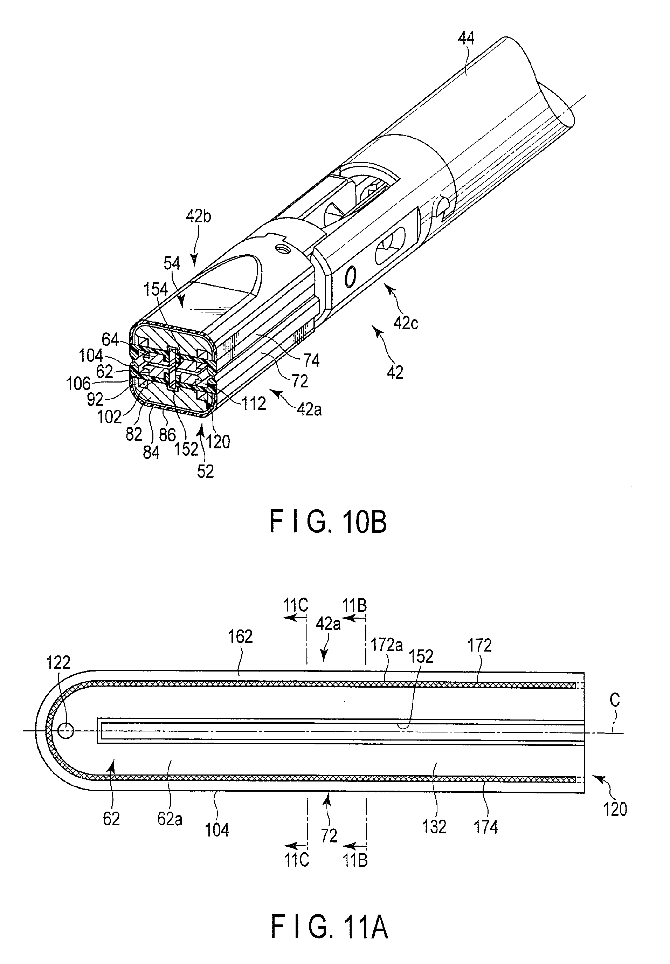

As shown in FIG. 11A to FIG. 11C, in this modification, a meshed member (living tissue support portion) 172 having a large number of openings 174 which allow the space 112 and the depressions 92 to be in communication with each other is provided instead of the coupling portions (living tissue support portions) 106 and the openings 108 described in the first embodiment. Here, the meshed member 172 not only includes the openings 174 formed by braiding, but also includes a large number of straight openings 174. The openings 174 of the example shown in FIG. 11B and FIG. 11C are formed straight.

A surface 172a of the meshed member 172 on the side of the space 112 (the other treatment portion 42b) can support the peripheral tissues. The openings 174 of the meshed member 172 function as paths to allow the fluid to flow into the depressions 92 from the space 112. That is, the meshed member 172 is disposed to cover at least a part of the flow path 120 while maintaining an entrance (the openings 174) to allow the fluid to flow into the flow path 120, and can support the living tissue to prevent the peripheral tissues of the living tissue of the treatment target from coming into the far part (depressions 92) of the flow path 120. Here, the openings 174 are formed to be smaller than the width of the space 112 between the energy emitter 62 and the outer edge 104. The entrance of the fluid is thus formed with the narrow entrance and with the great depth (depth direction), and this can make it difficult for the fluid to come out of the flow path 120 once the fluid has flowed in the flow path 120. The meshed member 172 serves as a lid to confine the fluid in the flow path 120. Thus, the meshed member 172 further ensures that the fluid can be confined in the flow path 120.

The structures and functions are similar in other respects to the structures and functions described in the first embodiment, and are therefore not described here.

In connection with the components of the intermediary portion 72 between the base 102 and the outer edge 104, the coupling portions 106 and the openings 108 have been described in the first embodiment, and the meshed member 172 having the openings 174 has been described in the first modification of the first embodiment. However, the above-described other structures that allow the fluid to pass through the depressions 92 and that can support the peripheral tissues are also permitted.

### [Second Embodiment]

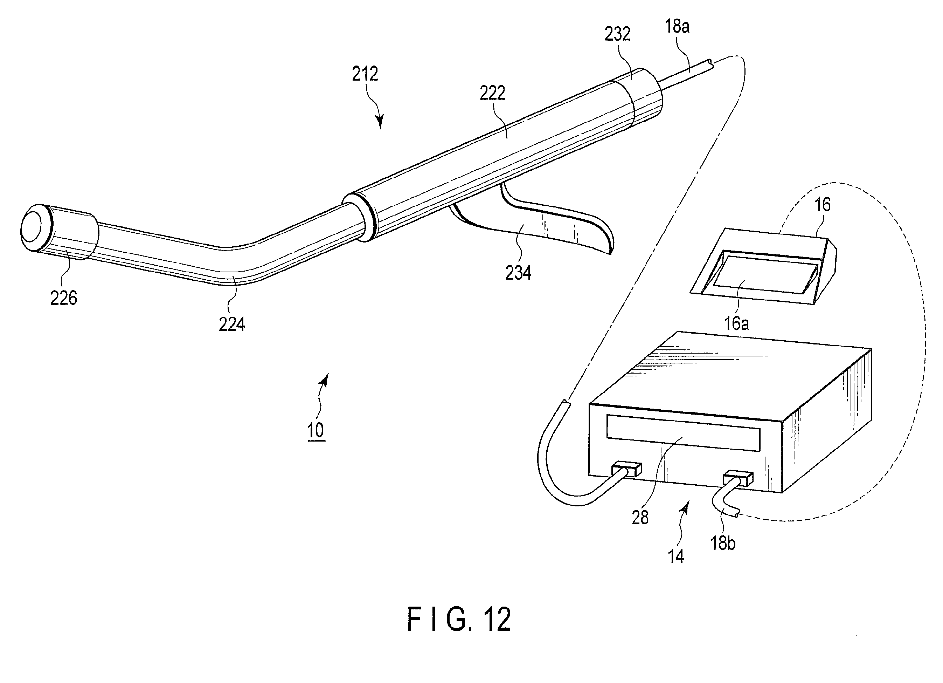

Now, the second embodiment is described with reference to FIG. 12 to FIG. 15B. This embodiment is a modification of the first embodiment including its modifications. The same components as the components described in the first embodiment or components having the same functions are provided with the same reference signs as much as possible, and are not described in detail.

Here, a treatment device (energy treatment device) 212 of a circular type to conduct a treatment through abdominal walls or outside abdominal walls is described as an energy treatment device by way of example. Although the bipolar type treatment device 212 is described in this embodiment, the return electrode R shown in FIG. 6B may be used to form a monopolar type energy treatment device.

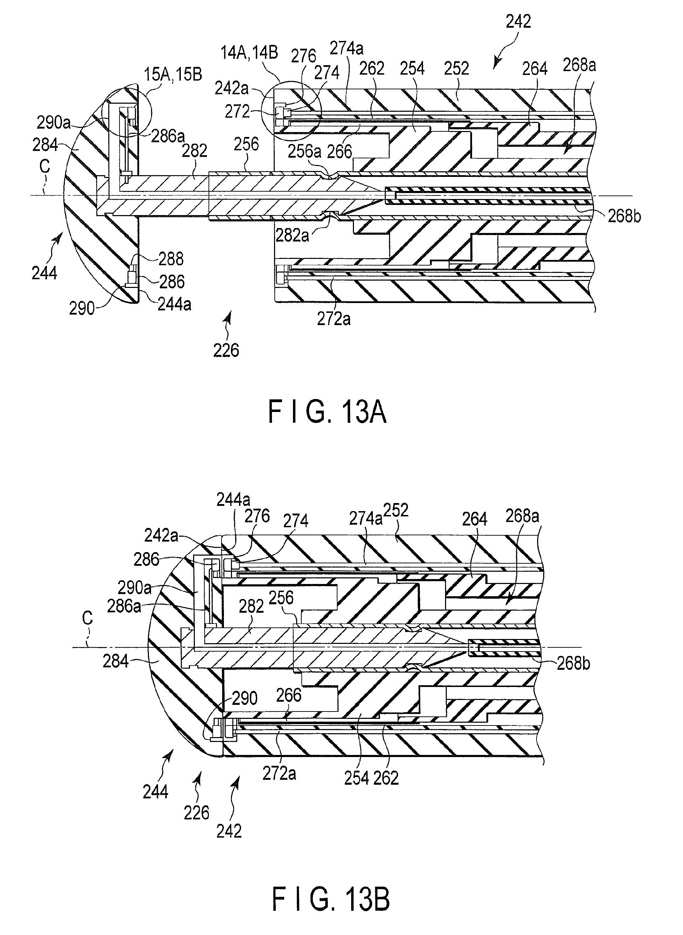

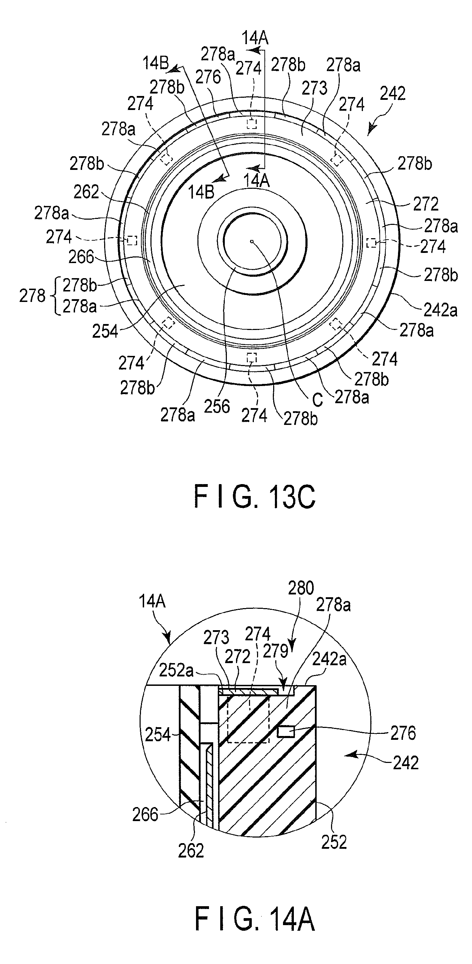

As shown in FIG. 13, a treatment system 10 using energy includes a treatment device (energy treatment device) 212, an energy source 14 which applies energy to the treatment device 212, and a foot switch 16.

The treatment device 212 includes a handle 222, a shaft 224, and an openable and closable treatment portion 226. The energy source 14 is connected to the handle 222 via a cable 18a.

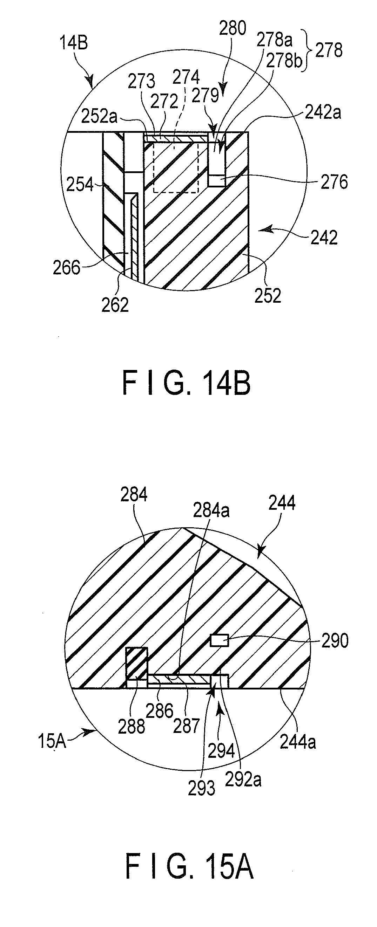

The handle 222 is provided with a holding portion open/close knob 232, and a cutter driving lever 234. The holding portion open/close knob 232 is rotatable relative to the handle 222. When the holding portion open/close knob 232 is rotated, for example, clockwise relative to the handle 222, a later-described separation side treatment portion (separation side grasp portion) 244 of the treatment portion 226 separates from a body side treatment portion (body side grasp portion) 242 (see FIG. 14A). When the holding portion open/close knob 232 is rotated counterclockwise, the separation side treatment portion 244 comes close to the main body side treatment portion 242 (see FIG. 14B).

As shown in FIG. 13, the shaft 224 is cylindrically formed. The shaft 224 is moderately curved in consideration of insertability into a living tissue. It should be understood that the shaft 224 that is formed straight is also preferable.

The treatment portion 226 is provided at the distal end of the shaft 224. As shown in FIG. 14A and FIG. 14B, the treatment portion 226 includes the main body side treatment portion (first holding member, first jaw) 242 formed at the distal end of the shaft 224, and the separation side treatment portion (second holding member, second jaw) 244 that is attachable to and removable from the main body side treatment portion 242. When the separation side treatment portion 244 is closed relative to the main body side treatment portion 242, outer edges 242a and 244a of the main body side treatment portion 242 and the separation side treatment portion 244 are in close contact to face each other or are in abutment with each other. Thus, the annular outer edge 242a faces the annular outer edge 244a, and form a holding portion (second holding portion) to hold peripheral tissues of the living tissue of the treatment target.

As shown in FIG. 14A and FIG. 14B, the main body side treatment portion 242 includes a cylinder 252, a frame 254, and an electric conduction pipe 256. The cylinder 252 and the frame 254 have an insulating property. The cylinder 252 is coupled to the distal end of the shaft 224. The frame 254 is provided to be fixed to the cylinder 252.

The central axis of the frame 254 is open. The electric conduction pipe 256 is provided in the open central axis C of the frame 254 movably within a predetermined range along the central axis C of the frame 254. When the holding portion open/close knob 232 is rotated, the electric conduction pipe 256 is movable within the predetermined range because of the function of, for example, a ball screw (not shown), as shown in FIG. 14A and FIG. 14B. A diametrically inwardly protruding protrusion 256a is formed in the electric conduction pipe 256 so that a connect portion 282a of a later-described electric conduction shaft 282 of the separation side treatment portion 244 can engage with the protrusion 256a.

As shown in FIG. 14A to FIG. 14C, a cutter guide groove (space) 266 is formed between the cylinder 252 and the frame 254. A cylindrical cutter 262 is provided in the cutter guide groove 266. The proximal end of the cutter 262 is connected to the distal end of a cutter pusher 264 provided inside the shaft 224. The cutter 262 is fixed to the outer circumferential surface of the cutter pusher 264. Although not shown, the proximal end of this cutter pusher 264 is connected to the cutter driving lever 234 of the handle 222. Thus, when the cutter driving lever 234 of the handle 222 is operated, the cutter 262 moves via the cutter pusher 264.

A first fluid ventilation path (fluid path) 268a is formed between the cutter pusher 264 and the frame 254. A fluid release opening (not shown) to discharge the fluid which has passed through the first fluid ventilation path 268a is formed in the shaft 224 or the handle 222.

As shown in FIG. 14A and FIG. 14B, the distal end of the cylinder 252 is made of a material having electric insulation and heat resistance. That is, an insulating portion 252a is formed at the distal end of the cylinder 252. Although the insulating portion 252a is integral with the cylinder 252 in this embodiment described, the insulating portion 252a may be separate from the cylinder 252. A first high-frequency electrode 272 and heat generating components 274 are provided as output components and energy emitters at the distal end of the cylinder 252.

The first high-frequency electrode 272 is provided outside the cutter guide groove 266 which is provided with the cutter 262. The first high-frequency electrode 272 is annularly formed in the same manner as the cutter guide groove 266. The distal end of a first electric conduction line 272a is fixed to the first high-frequency electrode 272. The first electric conduction line 272a is connected to the cable 18a via the main body side treatment portion 242, the shaft 224, and the handle 222.

As shown in FIG. 14A to FIG. 14C, the heat generating components 274 are fixed to the rear surface of the first high-frequency electrode 272 at appropriate intervals. The distal end of a heater electric conduction line 274a is fixed to the heat generating components 274. The heater electric conduction line 274a is connected to the cable 18a via the main body side treatment portion 242, the shaft 224, and the handle 222.

It is also preferable to use one or more plate-shaped heaters as the heat generating components 274.

An annular fluid release groove 276 is formed outside the first high-frequency electrode 272. The fluid release groove 276 is in communication with the first fluid ventilation path 268a. The above-mentioned outer edge 242a is formed outside the fluid release groove 276 at a position projecting from the first high-frequency electrode 272. That is, the outer edge 242a of the main body side treatment portion 242 is closer to a later-described head portion 284 of the separation side treatment portion 244 than the surface of the first high-frequency electrode 272. Thus, an edge (uppermost end) 243a on the inner side (the side close to the first high-frequency electrode 272) of the outer edge 242a serves as a barrier (dam) to prevent fluid such as vapor from escaping to the outside of the fluid release groove 276.

As shown in FIG. 13C, an annular support portion (living tissue support portion) 278 which supports the living tissue and which prevents the living tissue from coming into the fluid release groove 276 is formed between the first high-frequency electrode 272 and the outer edge 242a of the main body side treatment portion 242, that is, in the fluid release groove 276. In the support portion 278, projections 278a diametrically inwardly projecting from the outer edge 242a, and openings (second channel) 278b adjacent to the projections 278a are alternately arranged along the circumferential direction. As shown in FIG. 14A, the projections 278a are formed integrally with the outer edge 242a, and are diametrically inwardly projecting from the inner circumferential surface of the outer edge 242a. The openings 278b shown in FIG. 14B are in communication with the cutter guide groove 266 and the first fluid ventilation path 268a through the fluid release groove 276.

A surface on the side close to the separation side treatment portion 244 of the surface of the projection 278a is located closer to the shaft 224 and the handle 222 than the surface of the outer edge 242a that is facing the outer edge 244a of the separation side treatment portion 244, and than a holding surface 273 of the first high-frequency electrode 272. That is, a space (third channel) 279 is formed between the electrode 272 and the outer edge 242a. The fluid which has flowed to the surfaces of the projections 278a close to the separation side treatment portion 244 then flows into the fluid release groove 276 from the adjacent openings 278b.

The space (third channel) 279 and the openings (second channel) 278b between the electrode 272 and the outer edge 242a, and the fluid release groove (first channel) 276 cooperate to form a flow path 280 of the fluid emanating from the living tissue.

The separation side treatment portion 244 includes the electric conduction shaft 282 having the connect portion 282a, and the head portion 284. The electric conduction shaft 282 is circular in section, and has one end tapered and the other end fixed to the head portion 284. The connect portion 282a is formed into a depressed groove which can engage with the protrusion 256a of the electric conduction pipe 256. The outer surface of the electric conduction shaft 282 other than the connect portion 282a is insulated by, for example, coating.

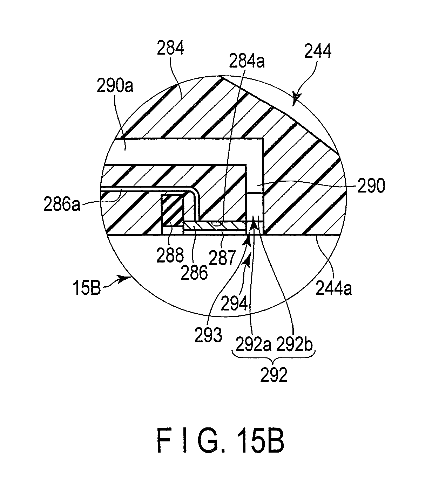

As shown in FIG. 15A and FIG. 15B, the position of the head portion 284 facing the distal end of the cylinder 252 is made of a material having electric insulation and heat resistance. That is, the head portion 284 forms an insulating portion 284a. Although the insulating portion 284a is integral with the head portion 284 in this embodiment, the insulating portion 284a may be separate from the head portion 284. A second high-frequency electrode 286 is provided in the head portion 284 as an output component or an energy emitter. That is, in the head portion 284, the second high-frequency electrode 286 is provided to face the first high-frequency electrode 272 of the main body side treatment portion 242. One end of a second electric conduction line 286a is fixed to the second high-frequency electrode 286. The other end of the second electric conduction line 286a is electrically connected to the electric conduction shaft 282.

The high-frequency electrodes 272 and 286 face each other, and are used as the holding surfaces (first holding portions) 273 and 287 of the living tissue of the treatment target. Thus, when high-frequency energy is applied to the electrodes 272 and 286 while the living tissue is held between the holding surfaces 273 and 287 of the electrodes 272 and 286, the living tissue can be denatured by the heat energy which has been generated. The electrode 272 is made of a material having satisfactory thermal conduction. Therefore, when heat is generated in the heat generating components 274, the heat (heat energy) is transmitted to the electrode 272, and the heat (heat energy) can be further transmitted to the living tissue which is in contact with the holding surface 273 of the electrode 272. Thus, the holding surfaces 273 and 287 also function as treatment surfaces of the living tissue. Although no heat generating component is disposed on the rear surface of the electrode 286 in this embodiment, the electrode 286 may be made of a material having satisfactory thermal conduction, a heat generating component may be disposed on the rear surface of the electrode 286, and the heat generated in heat generating component may be transmitted to the electrode 286.

A cutter receiver 288 is annularly formed on the inner side of the second high-frequency electrode 286 provided in the head portion 284 to receive the edge at the distal end of the cutter 262. On the other hand, a fluid release groove 290 is annularly formed on the outer side of the second high-frequency electrode 286. The above-mentioned outer edge 244a is formed outside the fluid release groove 290 at a position projecting from the surface of the second high-frequency electrode 286. That is, the outer edge 244a of the separation side treatment portion 244 is closer to the main body side treatment portion 242 than the surface of the second high-frequency electrode 286. Thus, an end 245a on the inner side (the side close to the second high-frequency electrode 286) of the outer edge 244a serves as a barrier (dam) to prevent fluid such as vapor from escaping to the outside of the fluid release groove 290.

Furthermore, the fluid release groove 290 is in communication with the head portion 284 and a fluid release path 290a of the electric conduction shaft 282. This fluid release path 290a is in communication with a second fluid ventilation path (fluid path) 268b of the electric conduction pipe 256. A fluid release opening (not shown) to discharge the fluid which has passed through the second fluid ventilation path 268b is formed in a shaft 204 or a handle 202.

An annular support portion 292 which supports the living tissue and which prevents the living tissue from coming into the fluid release groove 290 is formed between the second high-frequency electrode 286 and the outer edge 244a of the separation side treatment portion 244, that is, in the fluid release groove 290. In the support portion 292, projections 292a diametrically inwardly projecting from the outer edge 244a, and openings (second channel) 299b adjacent to the projections 292a are alternately arranged along the circumferential direction. As shown in FIG. 15A, the projections 292a are formed integrally with the outer edge 244a, and are diametrically inwardly projecting from the inner circumferential surface of the outer edge 244a. Openings 292b shown in FIG. 15B are in communication with the second fluid ventilation path 268b through the fluid release groove 290.

The surface of the projection 292a close to the main body side treatment portion 242 is located away from the shaft 224 and the handle 222 than the outer edge 244a and the holding surface 287. That is, a space (third channel) 293 is formed between the electrode 286 and the outer edge 244a. The fluid that has flowed to the surfaces of the projections 292a close to the main body side treatment portion 242 then flows into the fluid release groove 290 from the adjacent openings 292b.

The space (third channel) 293 and the openings (second channel) 292b between the electrode 286 and the outer edge 244a, and the fluid release groove (first channel) 290 cooperate to form a flow path 294 of the fluid emanating from the living tissue.

The electric conduction pipe 256 is connected to the cable 18a via the shaft 224 and the handle 222. Thus, the second high-frequency electrode 286 and the electric conduction pipe 256 are electrically connected to each other when the connect portion 282a of the electric conduction shaft 282 of the separation side treatment portion 244 engages with the protrusion 256a of the electric conduction pipe 256.

Next, the function of the medical treatment system 10 according to this embodiment is described.

The surgeon preliminarily operates the display section 28 (see FIG. 2 and FIG. 12) of the energy source 14 to set output conditions of the medical treatment system 10. More specifically, set electricity Pset [W] of high-frequency energy output, set temperature Tset [°C] of heat energy output, and thresholds Z1 and Z2 of impedance Z of the living tissue, for example, are set.

As shown in FIG. 13B, the treatment portion 226 and the shaft 224 of the surgical treatment device 212 are inserted into an abdominal cavity through, for example, an abdominal wall while the main body side treatment portion 242 is closed relative to the separation side treatment portion 244. The main body side treatment portion 242 of the surgical treatment device 212, and the separation side treatment portion 244 are put to face the living tissue of the treatment target.

The holding portion open/close knob 232 of the handle 222 is operated to grasp the living tissue of the treatment target with the main body side treatment portion 242 and the separation side treatment portion 244. In this case, the holding portion open/close knob 232 is rotated, for example, clockwise relative to the handle 222. Then the electric conduction pipe 256 is moved toward the distal end relative to the frame 254 of the shaft 224, as shown in FIG. 13A. Accordingly, space is formed between the main body side treatment portion 242 and the separation side treatment portion 244, and the separation side treatment portion 244 can be separated from the main body side treatment portion 242.

The living tissue to be treated is then disposed between the first high-frequency electrode 272 of the main body side treatment portion 242 and the second high-frequency electrode 286 of the separation side treatment portion 244. The electric conduction shaft 282 of the separation side treatment portion 244 is inserted into the electric conduction pipe 256 of the main body side treatment portion 242. In this condition, the holding portion open/close knob 232 of the handle 222 is rotated, for example, counterclockwise. Thus, the separation side treatment portion 244 is closed relative to the main body side treatment portion 242. In this way, the living tissue of the treatment target is held between the main body side treatment portion 242 and the separation side treatment portion 244. When the living tissue of the treatment target and its peripheral tissues are thus held by the treatment portions 242 and 244, the living tissue of the treatment target is disposed in close contact with the surfaces 273 and 287 of the electrodes 272 and 286. The peripheral tissues of the living tissue of the treatment target are held in close contact between the outer edges 242a and 244a.

When the living tissue is held by the treatment portions 242 and 244, the living tissue of the treatment target is held by the holding surfaces 273 and 287. Some of the peripheral tissues of the living tissue of the treatment target come into the space 279 of the flow path 280 of the main body side treatment portion 242, and some of the peripheral tissues of the living tissue of the treatment target come into the space 293 of the flow path 294 of the separation side treatment portion 244. The surfaces of the projections 278a support the provided peripheral tissues so that the peripheral tissues do not enter into the fluid release groove 276 from the adjacent openings 278b through the space 279. Thus, when the living tissue is held by the treatment portions 242 and 244, the peripheral tissues of the living tissue of the treatment target are held in a pressed state on the outer edges 242a and 244a of the treatment portions 242 and 244.

In this condition, the pedal 216a of the foot switch 216 is operated, and energy is applied to the first high-frequency electrode 272 and the second high-frequency electrode 286 from the energy source 14 via the cable 18a. Thus, the living tissue between the first high-frequency electrode 272 of the main body side treatment portion 242 and the second high-frequency electrode 286 of the separation side treatment portion 244 is heated by Joule heat.

As the living tissue is heated, fluid [liquid (blood) and/or gas (vapor)] is released from the living tissue. At the same time, the fluid released from the living tissue flows into the cutter guide groove 266 of the main body side treatment portion 242 and the flow path 280, and also flows into the flow path 294 of the separation side treatment portion 244. The fluid which has flowed into the cutter guide groove 266 of the main body side treatment portion 242 and the flow path 280 is, for example, sucked and passed to the shaft 224 from the cutter guide groove 266 through the first fluid ventilation path 268a. The fluid which has flowed into the flow path 294 of the separation side treatment portion 244 is, for example, sucked and passed to the shaft 224 from the head portion 284 and the fluid release path 290a of the electric conduction shaft 282 through the second fluid ventilation path 268b of the electric conduction pipe 256.

Here, from the holding surfaces 273 and 287 of the electrodes 272 and 286, the flow paths 280 and 294 are formed with a depth which is a combination of the thickness of the electrodes 272 and 286, the height (thickness) of the projections 278a and 292a diametrically inwardly projecting from the outer edges 242a and 244a of the treatment portions 242 and 244 in a direction parallel to the central axis C, and the depth of the fluid release grooves (depressions) 276 and 290. That is, the flow paths 280 and 294 are formed deeper than, for example, when the projections 278a and 292a are not provided and the thickness of the electrodes 272 and 286 and the fluid release grooves (depressions) 276 and 290 only cooperate to form the flow path. Thus, the volumes of the flow paths 280 and 294 can be increased without any change in the diametrical direction of the treatment portions 242 and 244. This further ensures that much of the fluid emanating from the living tissue can flow into the farthest parts of the fluid release grooves 276 and 290 of the flow paths 280 and 294 through the spaces 279 and 293 and the openings 278b and 292b of the flow paths 280 and 294. Out of the fluid emanating from the living tissue, the fluid which has flowed on the surfaces of the projections 278a and 292a through the spaces 279 and 293 of the flow paths 280 and 294 can flow into the farthest parts of the fluid release grooves 276 and 290 of the flow paths 280 and 294 through the openings 278b and 292b.

The flow paths 280 and 294 are formed deeper in a direction that intersects at right angles with the diametrical direction of the treatment portions 242 and 244 than, for example, when the flow paths only have the thickness of the electrodes 272 and 286 and the fluid release grooves (depressions) 276 and 290. This ensures that the fluid which has flowed into the far parts (the fluid release grooves (depressions) 276 and 290) in flow paths 280 and 294 does not easily come out of the flow paths 280 and 294. That is, the flow paths 280 and 294 are formed with narrow entrances and with a great depth (depth direction), and this can prevent the fluid from easily coming out of the flow paths 280 and 294 once the fluid has flowed in the flow paths 280 and 294. The projections 278a and 292a also serve to confine, in the flow paths 280 and 294, the fluid which has flowed into the fluid release grooves 276 and 290. Thus, the projections 278a and 292a can further ensure that the fluid is confined in the flow paths 280 and 294. Therefore, it is possible to maximally prevent the fluid from flowing out of the treatment portion 226.

Thus, while the fluid is being released from the living tissue, the fluid is kept flowing into the flow paths 280 and 294. Therefore, it is possible to prevent the thermal spread from being caused by the fluid released from the living tissue which has increased in temperature, and prevent parts that are not targeted for the treatment from being affected.

When the impedance Z is judged to be more than the threshold Z1, a signal is transmitted to the heat generating component driving circuit 26 from the controller 22. The heat generating component driving circuit 26 then supplies electricity to the heat generating components 274 so that the temperature of the heat generating components 274 will reach a preset temperature Tset [°C], for example, a temperature of 100 [°C] to 300 [°C]. Thus, the living tissue grasped between the electrodes 272 and 286 of the main body side treatment portion 242 and the separation side treatment portion 244 transmits heat to the first high-frequency electrode 272 from the heat generating components 274 by heat conduction. The living tissue which is in close contact with the first high-frequency electrode 272 is coagulated inward from the surface side by the heat.

The controller 22 then judges whether the impedance Z of the living tissue monitored by the high-frequency energy output circuit 24 is equal to or more than the preset threshold Z2. When the impedance Z is judged to be less than the threshold Z2, energy is kept applied to the heat generating components 274. On the other hand, when the impedance Z is judged to be equal to or more than the threshold Z2, the controller 22 sounds a buzzer from the speaker 30, and stops the output of the high-frequency energy and the heat energy. Thus, the treatment of the living tissue using the treatment system 10 is completed.

Thus, the living tissue is continuously (substantially annularly) denatured by the first and second high-frequency electrodes 272 and 286 and the heat generating components 274.

When the cutter driving lever 234 of the handle 222 is then operated, the cutter 262 projects from the cutter guide groove 266 of the main body side treatment portion 242, and then moves toward the cutter receiver 288 of the separation side treatment portion 244. Since the distal end of the cutter 262 has the edge, the treated living tissue is cut into an arc shape or a circular shape.

As described above, the following advantageous effects are obtained according to this embodiment.

The first high-frequency electrode 272 and the heat generating components 274 are annularly disposed in the main body side treatment portion 242, and the second high-frequency electrode 286 is annularly disposed in the separation side treatment portion 244 so that a treatment can be conducted. Thus, the living tissue between the main body side treatment portion 242 and the separation side treatment portion 244 can be substantially annularly treated.

Since the flow paths 280 and 294 are formed to be larger in the thickness direction (direction parallel to the central axis C) of the treatment portions 242 and 244, the volumes of the flow paths 280 and 294 can be increased without the increase in the diametrical direction of the treatment portions 242 and 244. Therefore, more of the fluid emanating from the living tissue by the treatment with energy can flow into the flow paths 280 and 294, and the emission of the heat to the outside of the treatment portions 242 and 244 through the peripheral tissues can be maximally prevented.

When the living tissue of the treatment target and its peripheral tissues are held between the treatment portions 242 and 244, the peripheral tissues can be held by the projections 278a and 292a, so that it is possible to prevent the peripheral tissues from coming into the fluid release grooves 276 and 290 through the openings 278b and 292b.

Although the projections 278a and 292a and the openings 278b and 292b are annularly and alternately arranged in the example described according to this embodiment, the meshed member described in the modification of the first embodiment may be used instead.

Although the several embodiments have been specifically explained with reference to the drawings, the present invention is not restricted to the foregoing embodiments, and it includes all embodiments carried out without departing from the gist of the invention.

## Claims

1. A treatment device which applies energy to a living tissue of a treatment target to treat the living tissue, the treatment device comprising a treatment portion, the treatment portion including:
a pair of jaws which are relatively openable and closable relative to each other and configured to hold and release the living tissue of the treatment target including peripheral tissues;
an energy emitter which is provided in at least one of the pair of jaws and which is configured to emit energy to the living tissue of the treatment target;
an insulating portion which is provided between the jaws and the energy emitter and which has electric insulation to cut off the flow of electricity from the energy emitter to the jaws;
an outer edge which is provided in the jaw;
a flow path formed by the cooperation of the side of one of the jaws that is provided with the energy emitter facing the other jaw, the energy emitter, the insulating portion, and the outer edges, fluid emanating from the living tissue of the treatment target by the energy emitted from the energy emitter flowing into the flow path; and
a support portion which is disposed in the flow path to cover at least a part of the flow path while maintaining an entrance to allow the fluid to flow into the flow path and which supports the living tissue to prevent the peripheral tissues of the living tissue of the treatment target from coming into the flow path.

2. The treatment device according to claim 1, wherein the flow path includes:
a first channel which is provided in one of the pair of jaws that is provided with the energy emitter and which is configured to collect the fluid emanating from the living tissue by the output of the energy from the energy emitter,
a second channel in which the support portion is formed and which is provided to cover the first channel so that a flow path in the first channel is ensured, and
a third channel formed between the energy emitter and the outer edges so that the flow paths in the first channel and the second channel are ensured.

3. The treatment device according to claim 1, wherein the support portion is formed to discretely cover the flow path.

4. The treatment device according to claim 1, wherein the support portion is formed to cover the flow path with a meshed member.

5. The treatment device according to claim 1, wherein
the jaw includes a main body in which the energy emitter is disposed, and a cover which is disposed on the side of the main body opposite to the side where the energy emitter is disposed and which covers the main body, and
a heat insulating layer is formed between the main body and the cover.
